Europäisches Patentamt

European Patent Office

Office européen des brevets

(11) Numéro de publication: **0 327 427**
**A1**

## (12) DEMANDE DE BREVET EUROPEEN

(21) Numéro de dépôt: **89400204.7**

(22) Date de dépôt: **25.01.89**

(51) Int. Cl.⁴: **A 61 B 5/04**

(30) Priorité: **26.01.88 FR 8800848**

(43) Date de publication de la demande:
**09.08.89 Bulletin 89/32**

(84) Etats contractants désignés:
**AT BE CH DE ES GB GR IT LI LU NL SE**

(71) Demandeur: **Gorny, Philippe**
**6, avenue Raphael**
**F-75016 Paris (FR)**

**Wierzbicki, Norbert**
**10, Villa Beauséjour**
**F-94300 Vincennes (FR)**

**Melloff, Pedro**
**15, rue Etienne Dolet**
**F-75020 Paris (FR)**

(72) Inventeur: **Gorny, Philippe**
**6, avenue Raphael**
**F-75016 Paris (FR)**

**Wierzbicki, Norbert**
**10, Villa Beauséjour**
**F-94300 Vincennes (FR)**

**Melloff, Pedro**
**15, rue Etienne Dolet**
**F-75020 Paris (FR)**

(74) Mandataire: **Leszczynski, André et al**
**CABINET NONY & CIE. 29 rue Cambacérès**
**F-75008 Paris (FR)**

(54) Procédé et dispositif de détection d'anomalies de l'activité cardiaque.

(57) L'invention est relative à un procédé de détection d'anomalies de l'activité cardiaque.

On détecte un signal de tension (V) représentatif de l'activité cardiaque, on traite électroniquement (1) ledit signal de manière à isoler les parties de signal $(V_1, V_2)$ de polarité positive et de polarité négative situées respectivement d'un côté et de l'autre de la ligne isoélectrique de la courbe d'activité cardiaque, on effectue séparément l'intégration (2a,2b) desdites parties de signal de polarité positive et desdites parties de signal de polarité négative sur une période correspondant à au moins un cycle d'activité cardiaque, on compare (4a,4b) chacune des valeurs d'intégration obtenues à une valeur de référence et on déclenche une alarme (5a,5b) si l'une au moins des comparaisons fournit un signal de différence.

EP 0 327 427 A1

## Description

### Procédé et dispositif de détection d'anomalies de l'activité cardiaque.

La présente invention est relative à un procédé et à un dispositif de détection d'anomalies de l'activité cardiaque.

Le coeur humain est fondamentalement une pompe à travers laquelle le sang est aspiré dans les orifices des oreillettes, puis remplit les ventricules et est ensuite pompé hors des ventricules vers les organes vitaux de l'organisme. Chaque cycle d'activité cardiaque est déclenché par une série d'évènements électriques qui se produisent dans un ordre spécifique.

Ces évènements électriques sont traditionnellement détectés et visualisés à l'aide d'un appareil d'électrocardiographie comportant des moyens, tels que des électrodes, pour fournir un signal de tension représentatif de l'activité cardiaque, signal qui est ensuite amplifié et filtré et reproduit sur un dispositif d'affichage, soit sous la forme d'un graphique sur papier, soit sur un écran.

Un électrocardiogramme conventionnel d'un battement de coeur normal comporte une onde P qui est la représentation de la dépolarisation du myocarde de l'orifice de l'oreillette, située au-dessus de la ligne isoélectrique, suivie par un complexe QRS qui reflète la dépolarisation du myocarde ventriculaire, situé pour l'essentiel au-dessus de la ligne isoélectrique, et enfin une onde T qui reflète la repolarisation ventriculaire et qui se situe également au-dessus de la ligne isoélectrique. Le segment ST est normalement sensiblement confondu avec la ligne isoélectrique.

Il existe de nombreux types d'anomalies de l'activité cardiaque, plus ou moins graves, qui se traduisent par des modifications plus ou moins importantes du tracé de l'électrocardiogramme.

Ainsi par exemple, les maladies coronariennes provoquent des anomalies de la repolarisation ventriculaire se traduisant par des sous-décalages ou des sus-décalages du segment ST par rapport à la ligne isoélectrique ou dans le cas d'angine de poitrine par une inversion de l'onde T qui se trouve alors entièrement en dessous de la ligne isoélectrique.

L'infarctus du myocarde aigü est pour sa part matérialisé par une onde Q importante en dessous de la ligne isoélectrique, une élévation du segment ST et une inversion de l'onde T.

Il existe déjà des dispositifs, utilisés principalement en milieu hospitalier pour détecter l'activité cardiaque, analysant par pointes en continu le tracé représentatif de l'activité, en convertissant sous forme numérique le signal analogique de tension fourni par les électrodes de détection, ces dispositifs étant équipés d'alarmes visuelles et/ou sonores pour signaler les variations du tracé par rapport à un tracé de référence programmé.

Il s'agit de dispositifs encombrants et très coûteux et qui sont donc généralement réservés à des applications cliniques.

Il existe par ailleurs des appareils portatifs d'enregistrement d'électrocardiogrammes réalisant également un traitement numérique du signal qui peut être ensuite converti en un signal analogique et reproduit sous forme graphique pour une lecture par le médecin traitant.

Un exemple d'un tel appareil est décrit dans le brevet français 82 16706 (2 533 816).

L'appareil décrit dans ce brevet peut être équipé d'un microprocesseur pouvant effectuer des traitements du signal numérique en vue de calculer la fréquence cardiaque et reconnaître et compter les principales arythmies.

Il s'agit là encore de dispositifs complexes et coûteux qui en outre ne permettent pas de détecter et de signaler instantanément à l'utilisateur les anomalies de l'activité cardiaque telles que celles mentionnées précédemment.

La présente invention se propose de fournir un procédé et un dispositif particulièrement simple fiable et économique, permettant précisément de détecter les principales anomalies de l'activité cardiaque et de déclencher un signal d'alerte avisant l'utilisateur lui-même d'une telle anomalie, en distinguant en outre les anomalies graves nécessitant un contrôle médical d'urgence et les anomalies peu graves ou passagères.

On doit comprendre que l'invention ne vise pas à fournir un procédé et un dispositif de diagnostic médical mais simplement un procédé et un dispositif d'alerte "grand public" utilisable par tous, notamment pendant ou au décours d'une pratique sportive, lors de l'apparition d'une douleur, ou à l'occasion d'un état d'anxiété, apte à signaler à l'utilisateur qu'une anomalie est constatée et qu'il doit alors consulter plus ou moins rapidement son médecin traitant.

Le procédé de détection d'anomalies de l'activité cardiaque selon l'invention se caractérise essentiellement par le fait que l'on détecte un signal de tension représentatif de l'activité cardiaque, que l'on traite électroniquement ledit signal de manière à isoler les parties de signal de polarité positive et de polarité négative situées respectivement d'un côté et de l'autre de la ligne isoélectrique de la courbe d'activité cardiaque, que l'on effectue séparément l'intégration desdites parties de signal de polarité positive et desdites parties de signal de polarité négative sur une période correspondant à au moins un cycle d'activité cardiaque, et de préférence sur une période comprise entre 15 secondes et 60 secondes, notamment de l'ordre de 30 secondes, que l'on compare chacune des valeurs d'intégration obtenues à une valeur de référence, et que l'on déclenche une alarme si l'une au moins des comparaisons fournit un signal de différence.

Dans un mode particulier de réalisation, les valeurs de référence sont établies à partir de l'activité cardiaque de l'utilisateur lui-même et sont obtenues en détectant un signal de l'activité cardiaque de l'utilisateur au repos, en traitant électroniquement le signal de manière à isoler les parties de signal de polarité positive et de polarité négative situées respectivement d'un côté et de l'autre de la ligne isoélectrique de la courbe d'activité cardiaque, en effectuant séparément l'intégration desdites parties de signal de polarité positive et desdites parties de signal de polarité négative sur une période correspondant à au moins un cycle d'activité cardiaque et de préférence sur une période comprise entre 15 secondes et 60 secondes et de préférence de l'ordre de 30 secondes, en mémorisant séparément les valeurs d'intégration obtenues, et en ajoutant à chaque valeur d'intégration une valeur prédéterminée.

Selon l'invention, il peut être prévu une alarme, sonore et/ou visuelle, associée à chacune des parties de signal situées de part et d'autre de la ligne isoélectrique de la courbe d'activité cardiaque, qui peuvent être déclenchées chacune lorsque la comparaison correspondante révèle une anomalie, ou bien l'on peut prévoir une alarme unique fonctionnant selon deux modes différents, par exemple sous la forme d'un fonctionnement continu ou intermittent, selon que l'une ou les deux comparaisons ont révélé une anomalie.

Ainsi par exemple dans le cas d'une anomalie à priori peu grave telle qu'une ischémie provoquant un sous-décalage du segment ST, la valeur d'intégration obtenue pour la partie de signal située en dessous de la ligne isoélectrique se trouvera augmentée et la comparaison effectuée permettre de déclencher l'alarme correspondante.

Dans le cas d'une anomalie grave, par exemple un infarctus du myocarde aigü, les deux valeurs d'intégration se trouveront augmentées ou, le cas échéant, l'alarme unique selon le mode particulier prescrit pour indiquer une anomalie grave, et une consultation médicale sans délai.

Le dispositif selon l'invention se compose d'un boîtier auquel sont raccordés des capteurs notamment des électrodes, généralement au nombre de deux ou de quatre, pour des mesures bipolaires, ces électrodes fournissant un signal de tension qui est traité à l'aide d'un premier circuit électrique conventionnel comportant des moyens d'amplification (amplificateur différentiel et amplificateur à gain fixe, et un ensemble de filtres comportant habituellement un filtre passe haut, un filtre passe bas, et un filtre de réjection de la fréquence du réseau électrique (selon le cas 50 ou 60 Hertz).

Le cas échéant, dans le cas d'un système à quatre électrodes, un multiplexeur est disposé entre les électrodes et le circuit comportant les moyens d'amplification et de filtrage.

Le dispositif selon l'invention comporte naturellement dans le boîtier une source d'alimentation en énergie électrique.

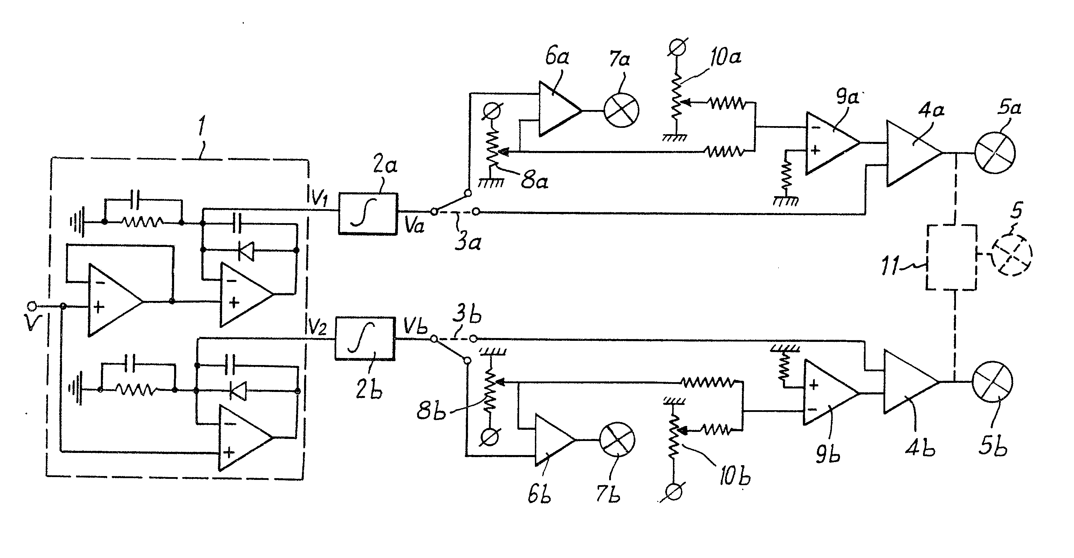

Le signal amplifié et filtré est ensuite traité électroniquement à l'aide d'un circuit illustré schématiquement sur la figure annexée.

Le signal de tension V représentatif de l'activité cardiaque provenant du circuit d'amplification et de filtrage (non représenté) est amené à un ensemble redresseur 1 de manière à isoler deux signaux $V_1$ et $V_2$ correspondant respectivement aux parties de signal de polarité positive et aux parties de signal de polarité néga       est-à-dire sur un tracé d'électrocardiogramme, les parties du tracé situées au-dessus et respectivement au-dessous de la ligne isoélectrique de la courbe d'activité cardiaque.

Chacun des signaux de tension $V_1$ et $V_2$ est intégré par rapport au temps sur une période par exemple de 30 secondes dans un intégrateur 2a et respectivement 2b fournissant à la sortie des signaux de tension Va et respectivement Vb qui correspondent aux aires des zones comprises entre la ligne isoélectrique et les parties de la courbe de l'électrocardiogramme situées respectivement au-dessus et au-dessous de la ligne isoélectrique.

Selon l'invention, chacun des signaux Va et Vb va maintenant être comparé à un signal de référence, en vue de détecter une anomalie éventuelle.

A cet effet il est prévu pour chacun des signaux Va et Vb un circuit de comparaison que l'on va décrire pour le signal Va, les composants de circuit électrique pour le signal Vb étant identiques et portant seulement l'indice b au lieu de l'indice a.

Le circuit de comparaison comporte un commutateur 3a dont la voie de commutation, représentée en trait interrompu, mène à l'entrée d'un comparateur 4a dont la sortie est reliée à une alarme 5a sonore ou visuelle, par exemple un témoin lumineux réalisé sur le boitier du dispositif.

La position en trait plein du commutateur 3a correspond à une phase d'étalonnage que l'on va maintenant décrire.

La valeur d'intégration Va obtenue par une mesure sur le sujet lui-même va être mémorisée dans le dispositif en tant que partie d'une valeur de référence à l'aide d'une partie de circuit comportant un comparateur 6a à la sortie duquel est prévu un élément indicateur 7a, par exemple une lampe, le comparateur 6a recevant le signal provenant de l'intégrateur a dans la position du commutateur 3a illustré en trait plein sur la figure. A la seconde entrée du comparateur 6a est relié le curseur d'un potentiomètre 8a associé à une source de tension de référence.

Afin de mémoriser un signal correspondant au signal d'intégration fourni par l'intégrateur 2a correspondant au patient lui-même, on ajuste le potentiomètre 8a de façon à ce que le signal envoyé par le potentiomètre au comparateur 6a corresponde au signal Va, l'actionnement du potentiomètre s'effectuant jusqu'au déclenchement de l'indicateur 7a.

La valeur d'intégration de référence est ensuite ajoutée à une valeur prédéterminée affichée en fonction de critères d'ordre médical à l'aide d'un second potentiomètre 10a relié à une tension de référence fixe, au travers d'un amplificateur opérationnel 9a, le signal de référence obtenu à la sortie de l'amplificateur 9a pouvant ainsi être comparé au signal d'intégration provenant de la mesure dans le comparateur 4a lorsque le commutateur 3a est dans la position représentée en trait interrompu.

Comme indiqué ci-dessus, les mêmes composants de circuit sont prévus pour le signal Vb intégrant les parties de la courbe situées en-dessous de la ligne isoélectrique de la courbe d'activité cardiaque.

Selon l'invention il peut être prévu deux alarmes séparées 5a et 5b associées  une  ie   p   ie

Va/Vb du signal V ou bien, comme schématisé en trait interrompu sur le dessin, il peut être prévu une alarme unique 5 associée à un circuit logique 11, l'alarme unique pouvant être actionnée selon un mode (par exemple un éclairement continu) lorsque l'un des comparateurs 4a ou 4b fournit un résultat de comparaison attestant d'une anomalie et dans un autre mode, par exemple un clignotement, lorsque les deux comparateurs 4a et 4b délivrent un signal d'anomalie, révélant ainsi une anomalie grave. A cet effet, le circuit logique 11 reçoit les signaux des comparateurs 4a et 4b et fournit le signal correspondant d'actionnement de l'alarme 5.

Il est bien évident que le schéma représenté n'est que l'illustration sous forme d'équivalents électriques des composants du dispositif, en particulier en ce qui concerne les potentiomètres. L'ensemble de la partie électronique du dispositif peut naturellement être réalisé sous forme de circuit intégré.

Bien que l'invention ait été décrite en liaison avec mode de réalisation particulier, il est bien évident qu'elle n'y est nullement limitée et qu'on peut lui apporter de nombreuses variantes et modifications sans pour autant sortir ni de son cadre ni de son esprit.

**Revendications**

1. Procédé de détection d'anomalies de l'activité cardiaque, caractérisé par le fait que l'on détecte un signal de tension (V) représentatif de l'activité cardiaque, que l'on traite électroniquement (1) ledit signal de manière à isoler les parties de signal ($V_1, V_2$) de polarité positive et de polarité négative situées respectivement d'un côté et de l'autre de la ligne isoélectrique de la courbe d'activité cardiaque, que l'on effectue séparément l'intégration (2a,2b) desdites parties de signal de polarité positive et desdites parties de signal de polarité négative sur une période correspondant à au moins un cycle d'activité cardiaque, que l'on compare (4a,4b) chacune des valeurs d'intégration obtenues à une valeur de référence et que l'on déclenche une alarme (5a,5b) si l'une au moins des comparaisons fournit un signal de différence.

2. Procédé selon la revendication 1, caractérisé par le fait que l'on effectue ladite intégration des parties de signal de polarité positive et desdites parties de signal de polarité négative sur une période comprise entre 15 secondes et 60 secondes, notamment de l'ordre de 30 secondes.

3. Procédé selon l'une quelconque des revendications 1 et 2, caractérisé par le fait que l'on établit les valeurs de référence à partir de l'activité cardiaque de l'utilisateur lui-même en détectant un signal de l'activité cardiaque de l'utilisateur au repos, en traitant électroniquement (1) le signal de manière à isoler les parties de signal de polarité positive et de polarité négative, en effectuant séparément l'intégration (2a,2b) desdites parties de signal de polarité positive et desdites parties de signal de polarité négative sur une période correspondant à au moins un cycle d'activité cardiaque, en mémorisant (6a,7a,8a,6b,7b,8b) séparément les valeurs d'intégration obtenues et en ajoutant (9a,10a,9b,10b) à chaque valeur d'intégration une valeur prédéterminée.

4. Procédé selon la revendication 3, caractérisé par le fait que ladite intégration desdites parties de signal de polarité positive et desdites parties de signal de polarité négative est effectuée sur une période comprise entre 15 secondes, et 60 secondes, et de préférence de l'ordre de 30 secondes.

5. Procédé selon l'une quelconque des revendications précédentes, caractérisé par le fait qu'il est prévu une alarme, sonore et/ou visuelle (5a,5b) associée à chacune des parties de signal ($V_1, V_2$) situées de part et d'autre de la ligne isoélectrique de la courbe d'activité cardiaque qui peuvent être déclenchées chacune lorsque la comparaison (4a,4b) correspondante révèle une anomalie.

6. Procédé selon l'une quelconque des revendications 1 à 4, caractérisé par le fait qu'il est prévu une alarme unique (5) fonctionnant selon deux modes différents, notamment sous la forme d'un fonctionnement continu ou intermittent, selon que l'une ou les deux comparaisons (4a,4b) ont révélé une anomalie.

7. Dispositif pour la mise en oeuvre du procédé selon l'une quelconque des revendications précédentes, comprenant un boitier muni d'une source d'alimentation électrique comportant un circuit pour amplifier et filtrer un signal représentatif de l'activité cardiaque et fourni par des capteurs, notamment des électrodes, caractérisé par le fait qu'il comporte un circuit de traitement électronique du signal comportant des moyens (1) pour isoler les parties de signal de polarité positive et les parties de signal de polarité négative situées respectivement d'un côté et de l'autre de la ligne isoélectrique de la courbe de l'activité cardiaque, des moyens d'intégration (2a,2b) pour intégrer séparément les parties de signal de polarité positive et les parties de signal de polarité négative sur une période correspondant à au moins un cycle d'activité cardiaque, des moyens de comparaison (4a,4b) de chacune des valeurs d'intégration obtenues à une valeur de référence et au moins une alarme (5,5a,5b) susceptible d'être déclenchée si une au moins des comparaisons fournit un signal de différence.

EP 0 327 427 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-3 707 959 (C.E. WILTON-DAVIES) * colonne 2, ligne 48 - colonne 3, ligne 28; figures 1,2 * --- | 1,3,5-7 | A 61 B 5/04 |
| A | WO-A-8 000 006 (J. ANDERSON et al.) * résumé; page 5, ligne 35 - page 6, ligne 35; figures 1,4 * --- | 1-4 | |
| A | EP-A-0 198 087 (K.K. KAISYA ADVANCE KAIHATSU KENKYUSO) * page 5, ligne 23 - page 7, ligne 16; figure 4 * ----- | 1,3,7 | |

DOMAINES TECHNIQUES
RECHERCHES (Int. Cl.4)

A 61 B 5/00

Le présent rapport a été établi pour toutes les revendications

| Lieu de la recherche | Date d'achèvement de la recherche | Examinateur |
|---|---|---|
| BERLIN | 13-04-1989 | WEIHS J.A. |